# EUROPÄISCHE PATENTSCHRIFT

(11) **EP 1 574 524 B1**
(45) Veröffentlichungstag und Bekanntmachung des Hinweises auf die Patenterteilung: **08.10.2008**
(21) Anmeldenummer: 05003704.3
(22) Anmeldetag: 21.02.2005
(51) Int. Cl.: C08F 2/02, C08F 2/46, A61K 6/083, A61K 6/093

(54) **Dentalwerkstoffe mit verbesserter Verträglichkeit**
Dental material with improved compatibility
Matériau dentaire ayant une compatibilité améliorée

(30) Priorität: 09.03.2004 DE 102004011497
(43) Veröffentlichungstag der Anmeldung: 14.09.2005
(73) Patentinhaber: Ivoclar Vivadent AG, 9494 Schaan (LI)
(72) Erfinder: Moszner, Norbert, Prof. Dr., FL - 9495 Triesen (LI); Fischer, Urs Karl, 9320 Arbon (CH); Burtscher, Peter Dr., 6830 Rankwell (AT); Angermann, Jörg, Dr., CH - 7320 Sargans (CH); Rheinberger, Volker, Dr., 9490 Vaduz (LI)
(74) Vertreter: UEXKÜLL & STOLBERG

(56) Entgegenhaltungen:
- GB-A- 2 280 905
- US-A- 4 966 934
- US-A- 5 081 164
- US-A- 5 180 756
- US-A- 5 276 068
- US-B1- 6 296 986

## Beschreibung

Die Erfindung betrifft Dentalwerkstoffe, die sich durch eine verbesserte Körperverträglichkeit auszeichnen.

Dentalmaterialien auf der Basis organischer Harze stellen in der Regel komplexe Gemische unterschiedlicher Komponenten dar, die neben polymerisierbaren Monomeren und/oder Oligomeren mindestens einen Initiator für die radikalische Polymerisation und darüber hinaus meist noch weitere Komponenten wie Polymerisationsbeschleuniger, Inhibitoren und/oder UV-Stabilisatoren enthalten. Hierbei handelt es sich häufig um niedermolekulare Verbindungen, die in toxikologischer Hinsicht problematisch sind. Bei der Härtung werden die verwendeten Monomere zum größten Teil kovalent in das sich bildende Polymernetzwerk eingebunden und damit daran gehindert, in das Umgebungsgewebe zu diffundieren. Die übrigen Komponenten werden jedoch nur physikalisch von dem Polymernetzwerk eingeschlossen und können so im Laufe der Zeit aus dem Dentalmaterial herausgewaschen werden, was unter dem Gesichtspunkt der Gewebeverträglichkeit unerwünscht ist.

Li et al., Macromol. Rapid Commun. 21 (2000) 590-594, beschreiben die Copolymerisation von 4,4'-Divinylbenzoylperoxid mit Methylmethacrylat und das Aufpfropfen von Butylacrylaten auf die Copolymere.

Dnebosky et al., J. Dent. Res. 54 (1975) 772-776, beschreiben N,N-substituierte Aminoethylmethacrylate, die sich als Beschleuniger für die benzoylperoxidkatalysierte Polymerisation von Methylmethacrylat eignen. Diese Aminoethylmethacrylate werden in die Polymerketten eingebaut, wodurch die Toxizität von Füllungskompositen verringert werden soll.

Tanzi et al., Clinical Materials 8 (1991) 131-136, offenbaren zwei ungesättigte, tertiäre Arylamine, nämlich N-Acryloyl-und Methacryloyl-N'-phenylpiperazin, die bei der durch Benzoylperoxid katalysierten radikalischen Polymerisation chemisch in das Polymernetzwerk eingebunden werden sollen.

Von der Firma Ciba Specialty Chemicals wird unter der Bezeichnung Ciba® Tinuvin® R 796 die chemische Verbindung 2-(2'-Hydroxy-5'-methacryloxyethylphenyl)-2H-benzotriazol vertrieben, die sich als copolymerisierbarer UV-Absorber beispielsweise zur Herstellung von Kontaktlinsen eignen soll.

Aus der DE OS 19 31 452 sind polymere Antioxidantien für Kunststoffe bekannt, die auf Monomeren mit sterisch gehinderten Phenolgruppen basieren.

Die DE OS 19 33 657 offenbart Acryloyloxyphenole und Polymerisate davon, die als Antioxidationsmittel insbesondere für Textilien geeignet sind.

Die US 5,276,068 betrifft Dentalmaterialien, die als Hauptkomponente Polycarbonat-Dimethacrylat-Kondensationsprodukte enthalten. Diese werden mit herkömmlichen Monomeren, Initiatoren, Antioxidantien und anderen Additiven kombiniert. Als geeignete Polymerisationsbeschleuniger für die Photopolymerisation werden unter anderem Dimethylaminoethylmethacrylat und Diethylaminoethylmethacrylat genannt.

Angiolini et al., Macromal. Chem. Phys. 201 (2000) 2646, haben polymere Photoinitiatoren und Aminbeschleuniger synthetisiert und ihr Polymerisationsverhalten untersucht. Die Verwendung einer polymeren Komponente führte zu einer Verzögerung der Polymerisation, bei der Verwendung beider polymerer Komponenten wurde eine deutliche Verringerung der Polymerisationsrate festgestellt, die auf sterische Hinderungen zurückgeführt wird. Die Polymerisationen wurden in Benzol als Lösungsmittel durchgeführt.

Bekannte Materialien enthalten immer einen mehr oder weniger großen Anteil von Komponenten, die bei der Härtung der Materialien nicht kovalent in das Polymernetzwerk eingebunden werden. Diese Komponenten können nach der Härtung der Materialien in benachbartes Körpergewebe einwandern und dort toxische Reaktionen auslösen.

Der Erfindung liegt die Aufgabe zugrunde, Dentalmaterialien bereitzustellen, die sich mittels Polymerisation aushärten lassen und welche nach der Härtung ein Minimum an Komponenten enthalten, die in Nachbargewebe diffundieren und unerwünschte Nebenreaktionen auslösen könnten.

Diese Aufgabe wird durch Werkstoffe gelöst, die ein radikalisch polymerisierbares organisches Bindemittel, mindestens einen Initiator für die radikalische Polymerisation, mindestens einen Beschleuniger für die radikalische Polymerisation und mindestens einen Inhibitor für die radikalische Polymerisation enthalten. Die Werkstoffe sind **dadurch gekennzeichnet, daß** Initiator, Beschleuniger und Inhibitor jeweils mindestens eine radikalisch polymerisierbare Gruppe aufweisen.

Es wurde gefunden, daß sich diese Massen problemlos durch radikalische Polymerisation härten lassen, wobei Materialien erhalten werden, die einen äußerst geringen Anteil an löslichen Komponenten enthalten. Dieses Ergebnis ist insofern überraschend, als der Stand der Technik eine deutliche Abnahme der Polymerisationsrate bei der Verwendung von Initiatoren und Beschleunigern mit polymerisierbaren Gruppen erwarten ließ, da beide Komponenten in das Polymernetzwerk eingebaut werden, was die Reaktionswahrscheinlichkeit durch sterische und kinetische Effekte reduziert (Angiolini, a.a.O.). Die gemeinsame Verwendung eines polymerisierbaren Initiators und eines polymerisierbaren Beschleunigers für die radikalische Polymerisation von Dentalwerkstoffen wurde bisher nicht beschrieben.

Als organische Bindemittel werden Monomere urd Oligomere mit radikalisch polymerisierbaren Gruppen verwendet. Unter radikalisch polymerisierbaren Gruppen werden hierin vorzugsweise ethylenisch ungesättigte Gruppen und insbesondere Meth)acryl-, Allyl-, Styryl-, Vinyl-, Vinyloxy- und/oder Vinylamin-Gruppen verstanden. Erfindungsgemäß eignen sich alle für einen Dentalwerkstoff brauchbaren Bindemittel, insbesondere Monomere und Oligomere, die mindestens eine ethylenisch ungesättigte Gruppe aufweisen. Derartige Monomere und Oligomere können allein oder in Mischung eingesetzt werden. Vorzugsweise enthält das Bindemittel mindestens ein polyfunktionelles Monomer oder Oligomer, d.h. ein Monomer oder Oligomer mit zwei oder mehr, vorzugsweise drei oder mehr und ganz besonders bevorzugt vier oder mehr polymerisierbaren ethylenisch ungesättigten Gruppen. Monomere und Oligomere mit zwei oder mehr radikalisch polymerisierbaren Gruppen wirken bei der Polymerisation als Vernetzer. Monomere oder Oligomere mit nur einer radikalisch polymerisierbaren Gruppe werden als monofunktionelle Monomere oder Oligomere bezeichnet.

Als Vernetzer eignen sich besonders Polysiloxane mit polymerisierbaren ethylenisch ungesättigten Gruppen und insbesondere (meth)acrylatmodifizierte Polysiloxane, die durch hydrolytische Kondensation z.B. von entsprechenden (meth)acrylatgruppenhaltigen Silanen zugänglich sind. Besonders bevorzugt sind Kondensate, die keine nichtfunktionalisierten Silanbausteine enthalten, d.h. Polysiloxane bei denen jede Siloxan-Wiederholungseinheit mindestens eine, vorzugsweise zwei oder drei polymerisierbare ethylenisch ungesättigte Gruppen aufweist. Diese Polysiloxane zeichnen sich durch eine hohe Funktionalität, d.h. eine hohe Anzahl polymerisationsfähiger Gruppen aus. Auf Grund der hohen Funktionalität findet ein praktisch vollständiger Einbau der Polysiloxane in das ausgehärtete Dentalmaterial statt. Darüber hinaus zeichnen sich die Polysiloxane auch durch eine geringe Löslichkeit in Wasser oder wäßrigen Lösungen aus, so daß auch geringste nicht polymerisierte Anteile aus dem Dentalmaterial unter oralen Bedingungen nicht löslich und damit auch nicht auswaschbar sind.

Für die Herstellung der polymerisierbaren Polysiloxane geeigneten (Meth)acrylsilane sind kommerziell zugänglich, wie z.B. 3-(Methacryloyloxy)propyltrimethoxysilan (**MEMO**)**,** oder lassen sich z.B. durch Umsetzung von Glycerindimethacrylat mit 3-Isocyanatopropyltriethoxysilan (**DMAURS,** EP 0 618 242 A2) oder

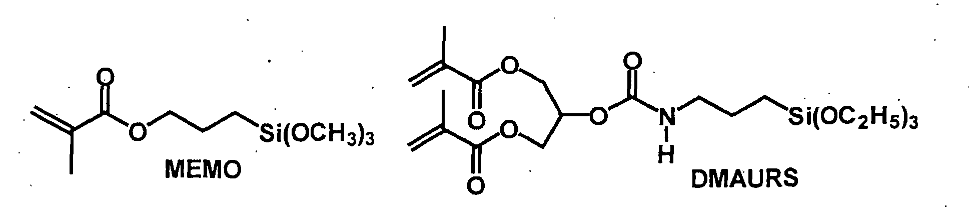

3-(Methyldiethoxysilyl)-propylbernsteinsäureanhydrid (**DMBES,** DE 44 16 857 C1) oder mit Glutarsäureanhydrid und anschließende Reaktion mit 3-Aminopropyltriethoxysilan **(DMAGAMS,** DE 199 03 177 C2) einfach herstellen, wobei in den angegebenen Veröffentlichungen auch die Herstellung der (meth)acrylatmodifizierten Polysiloxane durch hydrolytische Kondensation der (meth)acrylatgruppenhältigen Silane beschrieben ist.

Vorteilhaft, besonders für Komposite, sind auch die in der DE 199 03 177 C2 beschriebenen methacrylatgruppenhaltigen Polysiloxane, die aus Silanen hergestellt werden, bei denen die hydrolytisch kondensierbare Trialkoxysilylgruppe über eine flexible Aminoalkylgruppe mit den polymerisationfähigen Methacrylatgruppen verbunden ist (z.B. **DMAMS),** da sich die entsprechenden Polysiloxane **PK-DMAMS** durch eine vergleichsweise niedrige Viskosität auszeichnen. Im Falle von Kompositen kann so ein hoher Füllgrad erreicht werden.

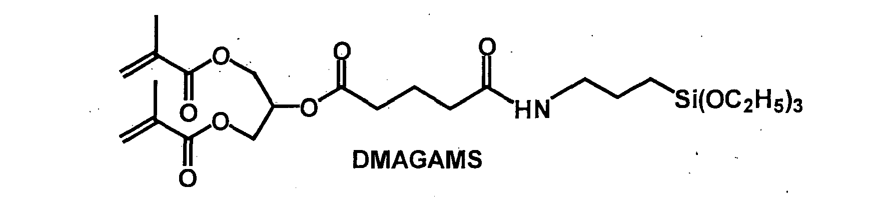

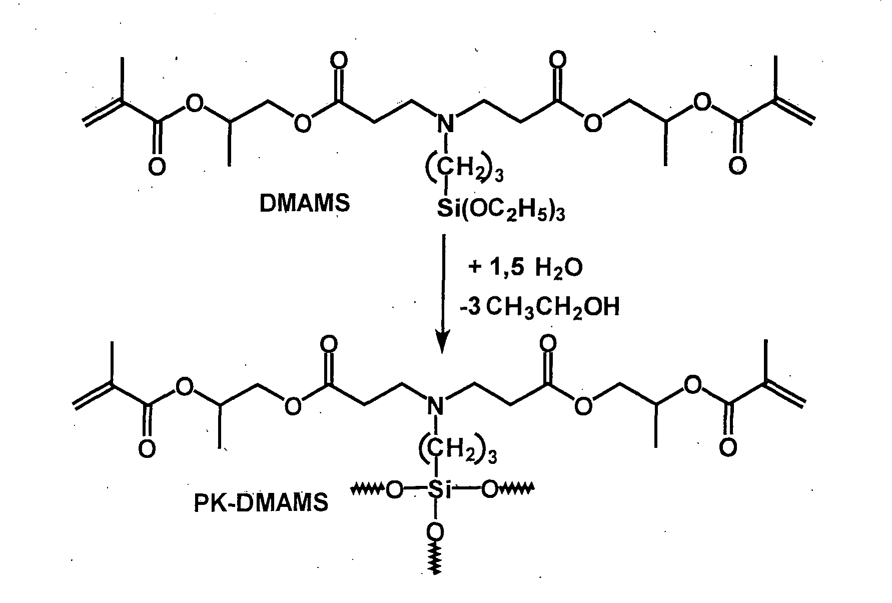

Weitere bevorzugte Bindemittel sind mit ethylenisch ungesättigten Gruppen modifizierte hyperverzweigte Polymere, sogenannte Dendrimere. Dendrimere sind dreidimensionale, hochgeordnete oligomere und polymere Verbindungen, die ausgehend von kleinen Initiator-Molekülen durch eine sich ständig wiederholende Reaktionsfolge synthetisiert werden (vgl. DE 44 43 702).

Erfindungsgemäß bevorzugte Dendrimere werden durch Reaktion von Hydroxyl- oder Aminogruppenhaltigen Initiatormolekülen mit Vinylcyaniden, wie beispielsweise Acryl- oder Methacrylnitril erhalten (Propylenimin-Deridrimere). Geeignete Propylenimin-Dendrimere und Verfahren zu deren Herstellung werden in der WO93/14147 beschrieben. Weitere Gruppen bevorzugter Dendrimere stellen die Polyether/Polythioether- (A.B. Padias et al.; Polym. Prepr. Am. Chem. Soc., Div. Polym. Chem. **30** (1989) 119), die Polyester-(WO93/17060), die Polyphenylenamid- (S.C.E. Backson et al.; Macromol. Symp. **77** (1994) 1) sowie die Polyphenylenester-Dendrimere (K.L. Wooley et al., Polymer Journal **26** (1994) 187) dar. Besonders bevorzugt sind Dendrimere, die eine sphärische Struktur aufweisen. Zudem sind Dendrimere der 4. oder einer höheren Generation erfindungsgemäß besonders geeignet.

Die Dendrimere weisen vorzugsweise ethylenisch ungesättigte Endgruppen auf. Als Endgruppen werden die reaktiven Gruppen der letzten Reaktandengeneration bezeichnet. Die Synthese von Dendrimeren mit polymerisationsfähigen Endgruppen erfolgt durch die aus der organischen Chemie bekannten Umsetzungen der o.g. Dendrimere mit geeigneten Monomer-Reagenzien. Besonders geeingete Ausgangsmaterialien sind Dendrimere mit Carboxyl-, Hydroxyl-und/oder Aminoendgruppen. Methacrylsäurechlorid und Isocyanatoethylmethacrylat sind zur Umsetzung hydroxy- oder aminofunktionalisierter Dendrimere und 2-Hydroxyethylmethacrylat zur Umsetzung carboxylgruppenhaltiger Dendrimere bevorzugt. Zur Umsetzung von aminogruppenhaltigen Dendrimeren ist die Michael-Reaktion mit Acryloyloxyethylmethacrylat (AEMA) besonders bevorzugt. Die Michael-Reaktion erfolgt selektiv an der Acrylät-Doppelbindung, während die Methacrylat-Doppelbindung als polymerisationsfähige Gruppe erhalten bleibt. Derartige Dendrimere und deren Herstellung werden in der DE 44 43 702 offenbart.

Eine weitere Gruppe erfindungsgemäß geeigneter polymerisierbarer Dendrimere stellen Epoxid-Amin-Dendrimere dar, die ein Kernmolekül mit mindestens einer primären Aminogruppe, mindestens einer Thiolgruppe, mindestens einer Phenolgruppe, mindestens einer Carbonsäuregruppe oder mindestens zwei sekundären Amingruppen oder mit einer Kombinationen dieser Gruppen und ein verzweigendes Molekül enthalten. Derartige Dendrimere und deren Herstellung werden in der WO 98/24831 offenbart.

Außerdem sind erfindungsgemäß Silan-Dendrimere mit terminalen Alkenylgruppen bevorzugt, wie sie in der DE 197 36 665 beschrieben werden. Mischungen der genannten Dendrimere sind ebenfalls geeignet.

Die oben genannten Polysiloxane und Dendrimere zeichnen sich dadurch aus, daß sie eine hohe Anzahl polymerisierbarer, ethylenisch ungesättigter Gruppen pro Molekül aufweisen und dementsprechend unter hohem Monomerumsatz polymerisiert werden können.

Ganz bevorzugte Polysiloxane sind Polykondensate, die durch hydrolytische Polykondensation eines oder mehrerer der folgenden Silane zugänglich sind: Bis[2-(2-(methacryloyloxyethoxycarbonyl)ethyl)]-3-triethoxysilylpropylamin, Bis[2-(2(1)-(methacryloyloxypropoxycarbonyl)ethyl)]-3-triethoxysilylpropylamin, 1,3(2)-Dimethacryloyloxypropyl-[3-(3-triethoxysilylpropyl)aminocarbonyl]propionat, 1,3(2)-Dimethacryloyloxypropyl-[4-(3-triethoxysilylpropyl)aminocarbonyl]butyrat, 1,3(2)-Dimethacryloyloxypropyl-[4-(3-triethoxysilylpropyl)-N-methylaminocarbonyl]butyrat, 3-[1,3(2)-Dimethacryloyloxypropyl)-2(3)-oxycarbonylamido]-propyltriethoxysilan.

Ganz besonders bevorzugte Dendrimere sind Polypropylenimin-, Epoxidamin- und Silandendrimere, die mindestens 4 endständige polymerisationsfähige Gruppen aufweisen, vorzugsweise Vinyl-und/oder (Meth)acrylgruppen.

Weitere bevorzugte Bindemittel sind radikalisch polymerisierbare Vernetzermonomere, wie die bekannten mehrfunktionellen Acrylate bzw. Methacrylate, z.B. Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)-acrylat, sowie Butandioldi(meth)acrylat, 1,10-Decandioldi(meth)acrylat oder 1,12-Dodecandioldi(meth)acrylat.

Erfindungsgemäß sind Bindemittel bevorzugt, die ausschließlich radikalisch polymerisierbare Monomere und/oder Oligomere mit zwei oder mehr ethylenisch ungesättigten Gruppen enthalten. Gemäß einer besonders bevorzugten Ausführungsform werden Bindemittel eingesetzt, die mindestens ein radikalisch polymerisierbares Polysiloxan und/oder ethylenisch ungesättigtes Dendrimer enthalten, ganz besonders bevorzugt solche, die ausschließlich ein oder mehrere radikalisch polymerisierbare Polysiloxane und/oder ethylenisch ungesättigte Dendrimere enthalten.

Als Initiatoren für die radikalische Polymerisation werden erfindungsgemäß ein oder mehrere Initiatoren eingesetzt, die mindestens eine radikalisch polymerisierbare Gruppe pro Initiatormolekül enthalten.

Als Initiatoren eignen sich besonders alpha-Diketone, Azoverbindungen, Peroxide, Benzildimethylketale, Benzoinether, Dialkoxyacetophenone und Trimethylbenzoylphosphinoxide mit ethylenisch ungesättigte Gruppen. Zusammensetzungen, die einen Photoinitiator enthalten, sind bevorzugt.

Bevorzugte Photoinitiatoren sind polymerisationsfähige Campherchinonderivate, wie beispielsweise 10-Methacryloyloxycampherchinon **(MACC),** das durch Umsetzung von 10-Hydroxycampherchinon mit Methacrylsäurechlorid erhalten werden kann (L. Angiolini, 2000, Macromol. Chem. Phys., 201, 2646).

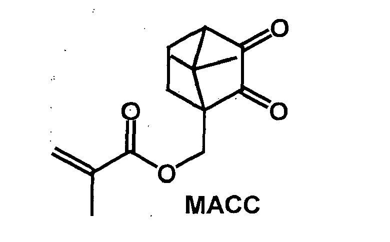

Derartige Campherchinonderivate werden vorzugsweise mit Aminen als Beschleuniger kombiniert.

Als thermische Initiatoren sind Peroxide und Azoverbindungen besonders geeignet. Ein bevorzugtes polymerisationsfähiges Peroxid ist zum Beispiel 4,4-Divinylbenzoylperoxid (**DVBPO),** das durch Umsetzung von 4-Vinylbenzoylchlorid mit Natriumperoxid zugänglich ist (Z. Li et al., 2000, Macromol. Rapid Commun., 590-594). Eine bevorzugte polymerisationsfähige Azoverbindung ist der Ester aus 2-Hydroxyethylmethacrylat und der 4,4'-Azobis-(4-cyan-valeriansäure) **ACVMA:**

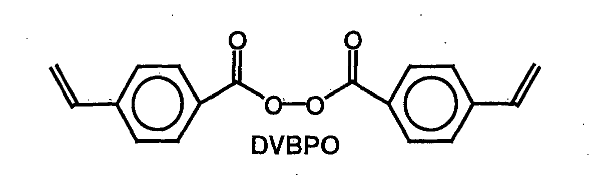

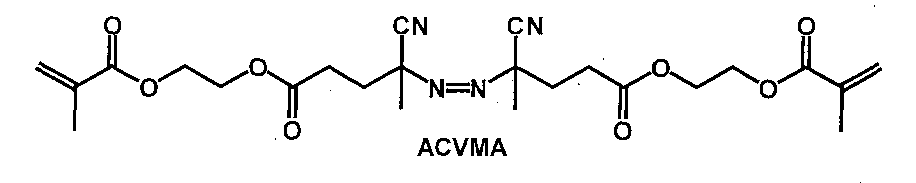

Derartige Azoverbindungen sind erhältlich, indem man geeignet funktionalisierte käufliche Azoverbindungen, wie z.B. Azobis(4-cyan-valerinsäure), mit geeignet funktionalisierten polymerisationsfähigen Verbindungen, im Falle der Azocarbonsäuren z.B. mit 2-Hydroxyethylmethacrylat in Gegenwart eines Kondensationsmittels, wie Dicyclohexylcarbodiimid, oder mit 2-Isocyanatoethylmethacrylat in Gegenwart eines Zinn-Katalysators umsetzt.

Bevorzugte polymerisierbare UV-Initiatoren leiten sich von Acylphosphinoxiden **(APO),** Dialkoxyacetophenonen oder Benzoinderivaten ab. Beispiele für polymerisationsfähige UV-Initiatoren sind das Styrol-Derivat **SAPO** (J. H. de Groot et al., 2001, Biomacromolecules, 2 1271), die Bisallylverbindung **DAA** (V.L. Mizyuk, et al. , 1994, Org. Chem. 30/4, 570) oder das Acrylat **BA** (P. Weiwei et al., 1998, Synthesis, 1298). Weitere Beispiele von monomeren Photoinitiatoren bzw. Sensibilisatoren sind in einer Übersicht von T. Corrales et al. (J. Photochem. Photobiol. A: Chem. 159 (2003) 103) enthalten.

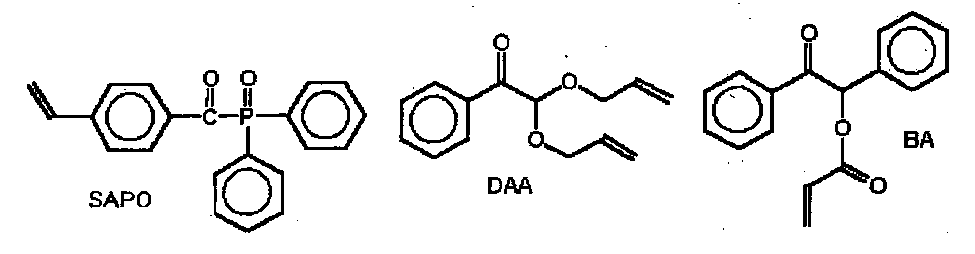

Als polymerisierbare Beschleuniger für die radikalische Polymerisation werden Reduktionsmittel eingesetzt, vorzugsweise Amine. Bevorzugte polymerisationsfähige Amine sind N-(2-Methacryloyloxyethyl)-N-methylanilin **(MAAMA),** N-(2-Methacryloyloxyethyl)-N-methyl-3,5-xylidin **(MAMA3,5X),** N-(2-Methacryloyloxyethyl)-N-methyl-p-toluidin **(MAAMpT),** N,N-Bis-(2-methacryloyloxyethyl)-p-toluidin **(DMAApT)** und N,N-Bis-(2-methacryloyloxyethyl)-3,5-xylidin **(DMAA3,5X)** (J. Dnebosky et al., 1975, J. Dent. Res. 54, 772-776).

Weitere bevorzugte polymerisationsfähige Amine, die als Beschleuniger für die radikalische Polymerisation geeignet sind, sind das kommerziell zugängliche 2-(Dimethylamino)ethylmethacrylat **(DMAEMA),** N-(2-Methacryloxyethyl)-N'-methylpiperazin **(MAMP)** oder N-(Methacrylamidomethyl)morpholin **(MAMMM)** (B. Vazquez, B. Levenfeld, J. San Roman, Polym. Intern. 1998, Role of amine activators on the curing parameters, properties and toxicity of acrylic bone cements, 46, 241-250, X. De Feng, Makromol. Chem., Macromol. Symp. 1992, The role of amine in vinyl radical polymerization, 63, 1-18).

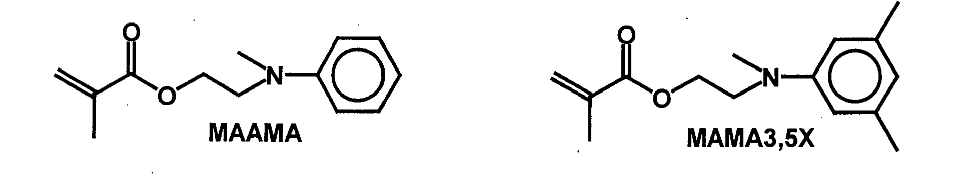

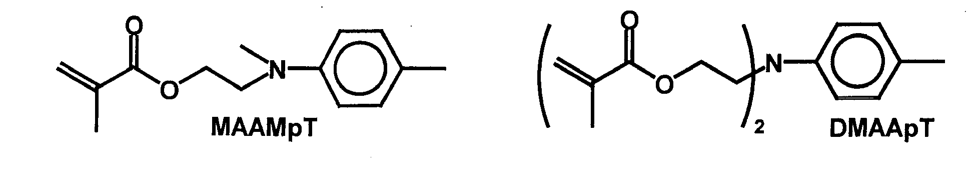

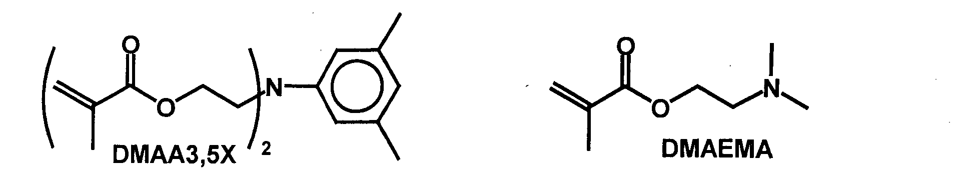

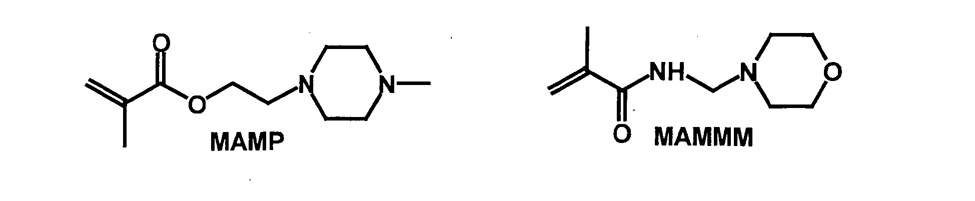

Werden die Aminbeschleuniger in Kombination mit Peroxiden eingesetzt, werden kalthärtende Werkstoffe erhalten.

Als Beschleuniger in Redoxsystemen kommen Barbitursäure- und Sulfinsäurederivate in Frage. Beispiele für polymerisationsfähige Derivate sind die Verbindungen **VOBA** (L. H. Cretscher et al., 1925, J. Amer. Chem. Soc. 47, 3083-3085) oder **SSA** (H. Kamogawa et al., 1976, Chem. Letters, 419-420):

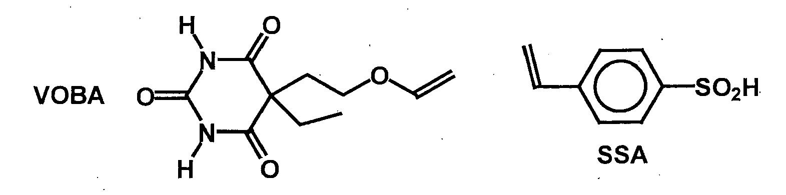

Neben den genannten Komponenten enthalten die erfindungsgemäßen Dentalwerkstoffe vorzugsweise auch mindestens einen polymerisationsfähigen UV-Absorber, d.h. einen UV-Absorber, der mindestens eine radikalisch polymerisierbare Gruppe aufweist. UV-Stabilisatoren dienen der Verbesserung der Farbstabilität der Materialien und verhindern z.B. eine Vergilbung unter Einwirkung ultravioletten Lichts, indem sie die energiereiche UV-Strahlung absorbieren und in Wärme umwandeln oder energiereiche Zustände deaktivieren. Geeignete polymerisationsfähige UV-Absorber werden beispielsweise in D. Bailey et al., 1976, J. Macromol. Sci.-Rev. Macromol. Chem. C14, 267-293, beschrieben. Bevorzugte polymerisierbare UV-Absorber sind Hydroxyphenylbenzotriazole, wie beispielsweise 2-(2"-Hydroxy-5'-methacryloyloxyethylphenyl-2-benzotriazol **(HMAEPBT),** das von der Firma Ciba Specialty Chemicals) unter der Bezeichnung Tinuvin® R796 kommerziell verfügbar ist.

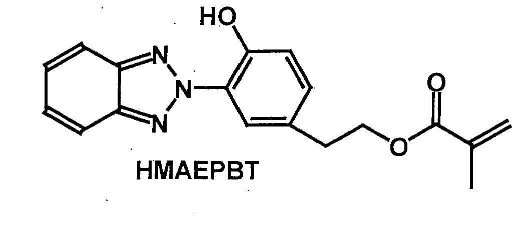

Außerdem enthalten die erfindungsgemäßen Werkstoffe mindestens einen radikalisch polymerisierbaren Inhibitor. Inhibitoren verhindern eine vorzeitig spontane Polymerisation, werden dementsprechend auch als Stabilisatoren bezeichnet und ermöglichen eine Lagerstabilität von ca. 2 Jahren. Man unterscheidet aerobe Inhibitoren, die nur in Gegenwart von Sauerstoff effektiv wirksam sind, und anaerobe Inhibitoren, die auch bei Abwesenheit von Sauerstoff wirksam sind. Bei selbsthärtenden Systemen gewährleisten Inhibitoren zudem eine ausreichende Verarbeitungszeit. In dieser sogenannten Inhibierungsperiode bilden die Inhibitoren mit den aus dem Initiator gebildeten Radikalen oder oligomeren Wachstumsradikalen inaktive Produkte. Erst wenn der Inhibitor verbraucht ist, beginnt die eigentliche Aushärtungsphase.

Als aerobe Inhibitoren sind polymerisationsfähige Phenolderivate bevorzugt. Besonders bevorzugte Beispiele hierfür sind 4-Hydroxyphenylacrylat **(HPhA)** (US 2, 675, 394) und 4-Methacryloyloxy-2,6-di-tert.-butylphenol **(MADtBPh)** (DE 19 33 657).

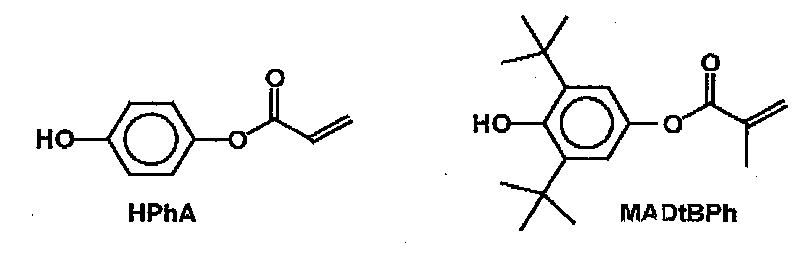

Bevorzugte anaerobe Inhibitoren sind polymerisierbare (Meth)acrylatderivate von 2,2,6,6-Tetramethyl-piperindin-1-oxyl-Radikal (TEMPO), wie beispielsweise cas N,N-Bis-(2-hydroxy-3-methacryloyloxypropoxy)-4-amino-2,2,6.6,-tetramethylpiperidin-1-oxyl-Radikal **(BMAP-TEMPO)** oder das N,N-Bis-(3-oxa-4-oxo-6-methacryloyloxyhexyl)-4-amino-2,2,6.6,-tetramethylpiperidin-1-oxyl-Radikal (BMAH-TEMPO) WO 01/60322) : 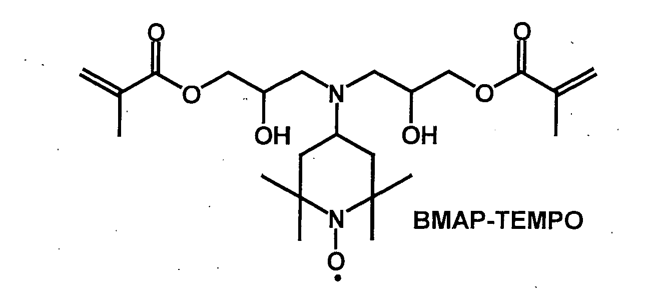 Erfindungsgemäß sind solche Dentalwerkstoffe besonders bevorzugt, die ausschließlich radikalisch polymerisationsfähige Komponenten, d.h. Initiatoren, Beschleuniger, ggf. UV-Stabilisatoren, ggf. Inhibitoren, enthalten, welche bei der Polymerisation in das Polymernetzwerk eingebunden werden können. Hierdurch wird der Anteil niedermolekularer Bestandteile, die nach der Härtung ausgewaschen oder in das Umgebungsgewebe diffundieren könnten, auf ein Minimum reduziert. Werkstoffe, die keine nicht radikalisch polymerisierbaren monomeren oder polymeren Bestandteile enthalten, sind besonders bevorzugt. Weiter bevorzugt sind Zusammensetzungen die kein Lösungsmittel enthalten. Unter Lösungsmittel werden hier bei Raumtemperatur flüssige Substanzen verstanden, die allein der Reaktionsführung oder der Erleichterung der Handhabung dienen, ohne in der fertig ausgehärteten Zusammensetzung noch vorhanden oder notwendig zu sein. Flüssige Monomere sind demgemäß keine Lösungsmittel in diesem Sinne.

Neben den genannten Bestandteilen können die erfindungsgemäßen Dentalwerkstoffe vorzugsweise auch Füllstoffe enthalten, die zur Verbesserung der mechanischen Eigenschaften dienen. Hierzu eignen sich organische oder anorganische Partikel oder Fasern. Bevorzugte anorganische partikuläre Füllstoffe sind amorphe, kugelförmige Materialien auf der Basis von Oxiden, wie ZrO₂ und TiO₂ oder Mischoxiden aus SiO₂, ZrO₂ und/oder TiO₂, nanopartikuläre oder mikrofeine Füllstoffe, wie pyrogene Kieselsäure oder Fällungskieselsäure sowie Makro- oder Minifüllstoffe, wie Quarz-, Glaskeramik- oder Glaspulver mit einer durchschnittlichen Teilchengröße von 0,01 bis 5 µm sowie röntgenopake Füllstoffe, wie Ytterbiumtrifluorid. Darüber hinaus können auch Glasfasern, Polyamid- oder Kohlenstoff-Fasern eingesetzt werden.

Je nach Art der Füllstoffe und der Anwendung liegt der maximale Füllgrad vorzugsweise zwischen 50 bis 90 Gew.-%, besonders bevorzugt 55-85 Gew.-%. Komposite, d.h. füllstoffhaltige Dentalwerkstoffe, werden nach der Partikelgröße und Zusammensetzung der Füllstoffe unterteilt in Makrofüller-Komposite, homogene und heterogene Mikrofüller-Komposite und Hybrid-Komposite. Makrofüller werden vorzugsweise durch Mahlen von Quarz, röntgenopaken Gläsern, Borosilikaten oder von Keramik gewonnen, sind rein anorganischer Natur und bestehen meist aus splitterförmigen Teilen einer mittleren Partikelgröße von ca. 0,4 bis 10 µm. Als Mikrofüller werden vorzugsweise pyrogenes SiO₂ oder Fällungskieselsäure eingesetzt, teilweise auch Mischoxide, z.B. SiO₂-ZrO₂, die durch hydrolytische Cokondensation von Metallalkoxiden (z.B. Tetraethoxysilan oder Tetrapropylzirkonat) zugänglich sind. Die Mikrofüller besitzen eine mittlere Partikelgröße von ca. 5 bis 100 nm und zeigen aufgrund ihrer großen spezifischen Oberfläche von 40 bis 300 m²/g einen starken viskositätserhöhenden Effekt. Heterogene Mikrofüller-Komposite enthalten sogenannte Mikrofüller-Komplexe. Beispiel hierfür sind splitterförmige vorpolymerisierte mikrogefüllte Komplexe, die z.B. durch Einarbeiten von pyrogenem SiO₂ in eine Harzmatrix, anschließender thermischer Härtung der Mischung und Mahlen des gefüllten Polymerisats zugänglich sind. Erfindungsgemäß sind Füllstoffe bevorzugt, die keine löslichen Komponenten enthalten.

Die Füllstoffe werden vor dem Einarbeiten in das Bindemittel vorzugsweise mit einem Haftvermittler oberflächenmodifiziert. Haftvermittler, die mindestens eine radikalisch polymerisierbare Gruppe aufweisen, sind bevorzugt. Besonders bevorzugte Haftvermittler sind Silane, insbesondere alpha- und gamma-Methacryloyloxypropyltrimethoxysilan. Werkstoffe, die mindestens 3 Gew.-% Füllstoff enthalten, sind bevorzugt.

Zusätzlich können die erfindungsgemäß eingesetzten Zusammensetzungen weitere Additive enthalten, wie z.B. Farbmittel (Pigmente) oder polymerisationsfähige, mikrobiocide Wirkstoffe. Pigmente werden zur Einstellung der Eigenfarbe bzw. der Transparenz zugesetzt. Bevorzugt sind anorganische Pigmente, vor allem Mischphasenpigmente auf Basis von Metalloxiden, z.B. TiO₂-Rutil mit Substitution von Titanionen durch Chrom bzw. Antimon (gelb) oder Ersatz der Metallionen im MgAl₂O₄-Spinell durch Kobalt (blau) oder Kupfer (schwarz) . Da solche Pigmente unlöslich sind, werden sie in der Regel die Biokompatibiltät des Dentalmaterials nicht beeinträchtigen.

Bevorzugte erfindungsgemäße Dentalwerkstoffe enthalten, abgesehen von Füllstoffen und Pigmenten, ausschließlich' Komponenten, die ethylenisch ungesättigte Gruppen aufweisen und so bei der Polymerisation in das Polymernetzwerk eingebunden werden. Füllstoffe und Pigmente sind in der Regel unlöslich und werden daher nicht aus dem Polymer ausgelaugt. Zur Verbesserung der mechanischen Eigenschaften sind die Füllstoffe jedoch bevorzugt mit Haftvermittlern behandelt, die ethylenisch ungesättigte Gruppen aufweisen, so daß auch diese in das Polymer integriert werden.

Besonders bevorzugt sind Dentalwerkstoffe, die
(a) 1 bis 50 Gew.-%, insbesondere 5 bis 40 Gew.-% Bindemittel, vorzugsweise Bindemittel mit zwei oder mehr polymerisierbaren Gruppen pro Bindemittelmolekül,
(b) 0,1 bis 5,0 Gew.-%, insbesondere 0,2 bis 2,0 Gew.-% radikalisch polymerisierbaren Initiator,
(c) 0,1 bis 5,0 Gew.-%, insbesondere 0,2 bis 2,0 Gew.-% radikalisch polymerisierbaren Beschleuniger,
(d) 0,01 bis 3,0 Gew.-%, insbesondere 0,05 b:s 2,0 Gew.-% Inhibitor, vorzugsweise radikalisch polymerisierbaren Inhibitor,
   und gegebenenfalls
(e) 0 bis 90, insbesondere 3 bis 80 Gew.-% Fühlstoff
enthalten.

Alle Angabe beziehen sich jeweils auf die Gesamtmasse des Werkstoffs.

Die erfindungsgemäßen Werkstoffe zeichnen sich dadurch aus, daß sie im Vergleich zu herkömmlichen Materialien nur einen sehr geringen Anteil an löslichen Komponenten enthalten, trotzdem aber innerhalb eines vergleichbaren Zeitraums gehärtet werden können. Dies ist erstaunlich, da im Hinblick auf die von Angiolini et al. a.a.O. veröffentlichten Ergebnisse eine deutlich verminderte Polymerisationsrate zu erwarten war, um so mehr als die erfindungsgemäßen Massen gemäß einer bevorzugten Ausführungsform nicht in einem Lösungsmittel polymerisiert werden, wodurch die von Angiolini et al. gefundenen sterischen Hinderungen weiter verstärkt werden sollten.

Die erfindungsgemäßen Dentalwerkstoffe eignen sich besonders als Füllungsmaterialien, Befestigungszemente und dentale Beschichtungsmaterialien. Darüber hinaus sind die Werkstoffe für die Herstellung von künstlichen Zähne, dentalen Prothesen, Inlays und Verblendmaterialien gut geeignet. Dentalmaterialien für intraorale Anwendungen sind bevorzugt.

Die Erfindung betrifft auch die Verwendung einer Zusammensetzung, die ein radikalisch polymerisierbares organisches Bindemittel, einen Initiator für die radikalische Polymerisation, einen Beschleuniger für die radikalische Polymerisation und einen Inhibitor für die radikalische Polymerisation enthält, wobei der Initiator, der Beschleuniger und der Inhibitor jeweils mindestens eine rad.,kalisch polymerisierbare Gruppe aufweisen, als Dentalmaterial oder zur Herstellung eines Dentalmaterials.

Die Erfindung wird im folgenden anhand von Beispielen näher erläutert.

### Ausführungsbeispiele

### Beispiel 1: Synthese des dimethacrylatgruppenhaltigen Polysiloxans OM-51

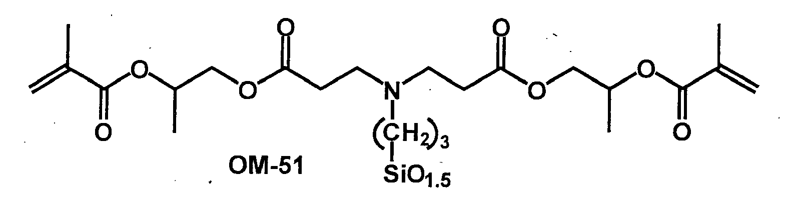

260 mmol (160,6 g) des Silans Bis- [ [methacryloyloxy) - propöxycarbonylethyl]-[triethoxysilylpropyl]-amin, das entsprechend der Literatur (N. Moszner, et al., 2002, Macromol. Mat. Eng., 287 339-347) hergestellt wurde, wurden in 380 ml EtOH gelöst. Die Hydrolyse des Silans erfolgte durch Zugabe von Wasser in Form einer 0,1 N NH₄F-Lösung (28,1 g). Nach 24 h Rühren bei Raumtemperatur wurden die flüchtigen Komponenten im Vakuum unter Einleitung von etwas Luft entfernt. Das entstandene viskose Harz (ca. 120 g **OM-51**) zeigte eine Viskosität _{η} von 8 Pas (23°C).

### Beispiel 2: Synthese des polymerisationsfähigen Aminbeschleunigers EMBO-MA

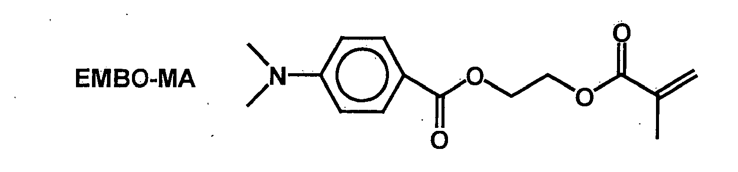

Analog zu N. Ono et al., 1978, Bull. Chem. Soc. Jpn., 51, 2401, wurde zu einer Lösung von 0,10 mol (17,1 g) 4-Dimethylaminobenzoesäure und 0,10 mol (15,8 g) 1,8-Diazabicyclo[5.4.0]undec-7-en in 240 ml Toluol langsam 0,10 mol (20,0 g) 2-Bromethylmethacrylat zugetropft und unter Rückfluß für 16 h erwärmt. Die Reaktionslösung wurde mit 1,0 N NaOH-Lösung, mit 1,0 N Salzsäure und schließlich mit Wasser gewaschen. Danach wurde die organische Phase über wasserfreiem Na₂SO₄ getrocknet, am Rotationsverdampfer eingeengt und über Nacht auskristallisieren gelassen. Es wurden 13,0 g (45,3 % der Theorie) weiße Kristalle an 2-(Methacryloyloxyethyl)-4-dimethylaminobenzoat **(EMBO-MA)** erhalten.

### Beispiel 3: Synthese eines polymerisationsfähigen. Campherchinon-Methacrylates MACC

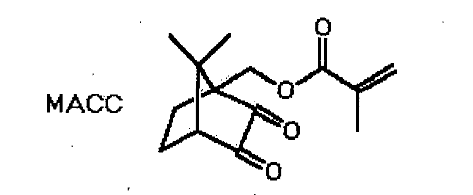

Analog zu L. Angiolini et al., 2000, Macromol. Chem. Phys. 201, 2646, wurde eine Mischung aus 10 mmol (1,82 g) 10-Hydroxycampherchinon, 10 mg 2,6-Di-tert.butyl-4-methylphenol (Inhibitor), 1,5 ml trockenem Triethylamin (TEA) und 50 ml absolutem 1,4-Dioxan unter Argon und Ausschluß von Tageslicht bei 0 °C zu einer gerührten Lösung von 11 mmol (1,15 g) Methacrylsäurechlorid, gelöst in 20 ml 1,4-Dioxan, zugetropft. Dann wurde 2 h bei Raumtemperatur und 2 h unter Rückfluß gerührt. Das Reaktionsgemisch wurde, vom gebildeten TEA-HCl abfiltriert, mit Diethylether verdünnt, mit 5 %-iger Natriumbicarbonatlösung und mit Wasser gewaschen. Nach Trocknen über wasserfreiem Na₂SO₄ wurde die Lösung am Rotationsverdampfer zur Trockene eingeengt und das feste Rohprodukt aus Toluol/n-Hexan (20:80 v/v) umkristallisiert. Es wurden 1,55 g (62 % der Theorie) an gelben Kristallen von 10-Methacryloyloxycampherchinon **MACC** erhalten.

### Beispiel 4: Synthese des polymerisationsfähigen Inhibitors MA-HQ

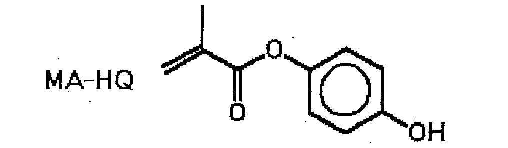

Analog zu DE 193 36 57 wurden zu 145 mmol (16,0 g) frisch umkristallisiertem Hydrochinon, gelöst in 150 ml wasserfreiem Tetrahydrofuran, unter Argon 140 mmol (21,0 g) Methacrylsäureanhydrid und anschließend 1,0 ml 96%-ige Schwefelsäure bei einer Temperatur < 25°C zugegeben. Die Reaktionsmischung wurde 16 h bei Raumtemperatur gerührt. Dann wurde der Ansatz in 800 ml Wasser gegossen, mit 2 N Natronlauge neutralisiert, für 2 h gerührt und das feste Rohprodukt abgesaugt. Anschließend wurde das Produkt mehrfach aus n-Hexan umkristallisiert und man erhielt 9,17 g (37 % der Theorie) weiße Kristalle an Hydrochinonmonomethacrylat **MA-HQ.**

### Beispiel 5: Komposit auf Basis von polymerisationsfähigen Matrixkomponenten

Es wurde eine Mischung der unten angegebenen Monomerkomponenten hergestellt. Diese wurde anschließend in einem Planeten-Kneter (Linde) mit dem Füllstoff zu einer homogenen Masse verarbeitet.

**Zusammensetzung der Monomermischung:**

| *Komponente* | *Anteil* |
|---|---|
| Polysiloxan OM51 | 49,26 Gew.-% |
| Urethandimethacrylat UDMA | 49,25 Gew.-% |
| Polymerisationsfähiges Campherchinon MACC | 0,50 Gew.-% |
| Polymerisationsfähiges Amin EMBO-MA | 0.85 Gew.-% |
| Polymerisationsfähiger Inhibitor MA-HQ | 0,14 Gew.-% |

**Zusammensetzung der Kompositpaste:**

| *Komponente* | *Anteil* |
|---|---|
| Monomermischung | 22,12 Gew.-% |
| Füllstoffmischung ^{a)} | 77,88 Gew.-% |

| | |
|---|---|
| ^{a)} Bariumglas 1.0 µm, silanisiert mit Methacryloyloxypropyltrimethoxysilan (67,88 Gew.-%), Sphärosil (14,42 Gew.-%), silanisiert mit Methacryloyloxypropyltrimethoxysilan (4,42 Gew.-%), Ytterbiumfluorid YbF₃ (13, 28' Gew. -%) | |

Aus der Kompositpaste wurden Prüfkörper (Scheiben: Höhe 2 mm, Durchmesser: 10 mm) bei einer Bestrahlungszeit von 2 mal 3 min. mit einem Spectramat (Ivoclar Vivadent AG) präpariert. Die Prüfkörper wurden anschließend mit Ethanol 3 Tage lang bei 37 °C erschöpfend extrahiert. In den Extrakten wurde der Anteil an nicht umgesetzten löslichen Komponenten durch Hochdruckflüssigkeitschromatographie (HPLC) ermittelt. Die quantitative Bestimmung erfolgte durch Auswertung der Integration der jeweiligen Peakflächen. Die Ergebnisse sind in Tabelle 1 angegeben.

### Beispiel 6: Komposit auf Basis von nicht polymerisierbaren Matrixkomponenten (Vergleich)

Analog zu Beispiel 5 wurde eine Mischung der unten angegebenen Monomerkomponenten hergestellt. Diese wurde anschließend in einem Planeten-Kneter (Linde) mit dem Füllstoff zu einer homogenen Masse verarbeitet.

**Zusammensetzung der Monomermischung:**

| *Komponente* | *Anteil* |
|---|---|
| Dimethacrylat TEGDMA | 15,00 Gew.-% |
| Dimethacrylat Bis-GMA | 35,00 Gew.-% |
| Urethandimethacrylat UDMA | 48,97 Gew.% |
| Campherchinon CC | 0,33 Gew.-% |
| Beschleuniger EMBO | 0.60 Gew.-% |
| Inhibitor MEHQ | 0,10 Gew.-% |

**Zusammensetzung der Kompositpaste:**

| *Komponente* | *Anteil* |
|---|---|
| Monomermischung | 22,12 Gew.-% |
| Füllstoffmischung ^{a)} | 77,88 Gew.-% |

| | |
|---|---|
| ^{a)} Zusammensetzung der Füllstoffmischung siehe Beispiel 5 | |

Auf die in Beispiel 5 beschriebene Weise wurden aus der Kompositpaste Prüfkörper präpariert und diese mit Ethanol erschöpfend extrahiert. Es wurden die gleichen molaren Mengen an Inhibitor, Beschleuniger und Initiator wie in Beispiel 5 verwendet. In Tabelle 1 ist das Analysenergebnis der Extrakte (nicht polymerisierte lösliche Komponenten) dargestellt.

**Tabelle 1**

| **Anteil an löslichen Komponenten in den Prüfkörpern** | | |
|---|---|---|
| **Komponente** | **Beispiel 5** [%]^{a)} | **Beispiel 6** (Vergleich) [%] ^{a)} |
| Monomer | 0,9 | 5,97 |
| Photoinitiator | 0,01 | 0,4 |
| Aminbeschleuniger | 1,2 | 17,5 |
| Inhibitor | 2,2 | 33,5 |

| | | |
|---|---|---|
| ^{a)} % lösliche Anteile bezogen auf die eingesetzte Menge | | |

Tabelle 1 zeigt, daß bei Verwendung von Matrixmaterialien auf der Basis von polymerisationsfähigen organischen Komponenten in einem Dentalmaterial bei identischen Härtungsbedingungen der Anteil an lösbaren Bestandteilen deutlich reduziert werden kann, was sich günstig auf die Biokompatibilität des Materials auswirkt.

## Patentansprüche

1. Dentalwerkstoff enthaltend ein radikalisch polymerisierbares organisches Bindemittel, mindestens einen Initiator für die radikalische Polymerisation, mindestens einen Beschleuniger für die radikalische Polymerisation und mindestens einen Inhibitor für die:radikalische Polymerisation, **dadurch gekennzeichnet, daß** Initiator, Beschleuniger und Inhibitor jeweils mindestens eine radikalisch polymerisierbare Gruppe aufweisen.

2. Dentalwerkstoff nach Anspruch 1, bei dem das Bindemittel Monomere und/oder Oligomere mit zwei- oder mehr radikalisch polymerisierbaren Gruppen enthält.

3. Dentalwerkstoff nach Anspruch 2, bei dem das Bindemittel mindestens ein Polysiloxan mit radikalisch polymerisierbaren Gruppen enthält.

4. Dentalwerkstoff nach Anspruch 2 oder 3, bei dem das Bindemittel mindestens ein Dendrimer enthält, das radikalisch polymerisierbare Gruppen aufweiset.

5. Dentalwerkstoff nach einem der Ansprüche 2 bis 4, bei dem das Bindemittel mindestens ein radikalisch polymerisierbares Vernetzermonomer enthält.

6. Dentalwerkstoff nach einem der Ansprüche 2 bis 5, bei dem das Bindemittel ausschließlich radikalisch polymerisierbare Monomere und/oder Oligomere mit zwei oder mehr radikalisch polymerisierbaren Gruppen enthält.

7. Dentalwerkstoff nach einem der vorhergehenden Ansprüche, der einen Photoinitiator enthält.

8. Dentalwerkstoff nach einem der vorhergehenden Ansprüche, der als Initiator mindestens ein alpha-Diketon eine Azoverbindung, ein Peroxid, ein Benzildimethylketal, einen Benzoinether, ein Dialkoxyacetophenon und/oder ein Trimethylbenzoylphosphinoxid enthält.

9. Dentalwerkstoff nach einem der vorhergehenden Ansprüche, der als Beschleuniger ein Amin, ein Barbitursäurederivat oder ein Sulfinsäurederivat enthält.

10. Dentalwerkstoff nach einem der vorhergehenden Ansprüche, der zusätzlich mindestens einen UV-Absorber enthält, der mindestens eine radikalisch polymerisierbare Gruppe aufweist.

11. Dentalwerkstoff nach einem der vorhergehenden Ansprüche, der zusätzlich ein Antioxidationsmittel enthält, das mindestens eine radikalisch polymerisierbare Gruppe aufweist.

12. Dentalwerkstoff nach einem der vorhergehenden Ansprüche, der einen UV-Stabilisator und einen Inhibitor enthält, die beide eine radikalisch polymerisierbare Gruppe aufweisen.

13. Dentalwerkstoff nach einem der vorhergehenden Ansprüche, der keine nicht radikalisch polymerisierbaren monomeren oder polymeren Bestandteile enthält.

14. Dentalwerkstoff nach einem der vorhergehenden Ansprüche, der kein Lösungsmittel enthält.

15. Dentalwerkstoff nach einem der vorhergehenden Ansprüche, der zusätzlich Füllstoff enthält.

16. Dentalwerkstoff nach Anspruch 15, bei dem der Füllstoff mit einem Haftvermittler oberflächenmodifiziert ist.

17. Dentalwerkstoff nach Anspruch 16, bei dem der Haftvermittler mindestens eine radikalisch polymerisierbare Gruppe aufweist.

18. Dentalwerkstoff nach einem der vorhergehenden Ansprüche, der zusätzlich ein oder mehrere Pigmente enthält.

19. Dentalwerkstoff nach einem der vorhergehenden Ansprüche, der, abgesehen von Pigmenten und Füllstoff, ausschließlich Komponenten enthält, die radikalisch polymerisierbare Gruppen aufweisen.

20. Dentalwerkstoff nach einem der vorhergehenden Ansprüche, der
(a) 1 bis 50 Gew.-%, insbesondere 5 bis 40 Gew.-% Bindemittel,
(b) 0,1 bis 5,0 Gew.-%, insbesondere 0,2 a bis 2,0 Gew.-% Initiator,
(c) 0,1 bis 5,0 Gew.-%, insbesondere 0,2 a bis 2,0 Gew.-% Beschleuniger,
(d) 0,01 bis 3,0 Gew.-%, insbesondere 0,05 bis 2,0 Gew.-% Inhibitor
enthält, jeweils bezogen auf die Gesamtmasse des Werkstoffs.

21. Dentalwerkstoff nach Anspruch 20, der zusätzlich (e) 0 bis 90, insbesondere 3 bis 80 Gew.-% Füllstoff enthält, bezogen auf die Gesamtmasse des Werkstoffs.

22. Verwendung einer Zusammensetzung, die ein radikalisch polymerisierbares organisches Bindemittel, einen Initiator für die radikalische Polymerisation, einen Beschleuniger für die radikalische Polymerisation und einen Inhibitor für die radikalische Polymerisation enthält, wobei der Initiator, der Beschleuniger und der Inhibitor jeweils mindestens eine radikalisch polymerisierbare Gruppe aufweisen, als Dentalmaterial oder zur Herstellung eines Dentalmaterials.

## Claims

1. Dental material, comprising a radically polymerisable organic binder, at least one initiator for the radical polymerisation, at least one accelerator for the radical polymerisation and at least one inhibitor for the radical polymerisation, **characterized in that** the initiator, the accelerator and the inhibitor each have at least one radically polymerisable group.

2. Dental material according to claim 1, wherein the binder contains monomers and/or oligomers with two or more radically polymerisable groups.

3. Dental material according to claim 2, wherein the binder contains at least one polysiloxane with radically polymerisable groups.

4. Dental material according to claim 2 or 3, wherein the binder contains at least one dendrimer which has radically polymerisable groups.

5. Dental material according to any one of claims 2 to 4, wherein the binder contains at least one radically polymerisable crosslinking monomer.

6. Dental material according to any one of claim 2 to 5, wherein the binder contains exclusively radically polymerisable monomers and/or oligomers with two or more radically polymerisable groups.

7. Dental material according to any one of the preceding claims, containing a photoinitiator.

8. Dental material according to any one of the preceding claims, which contains as initiator at least one α-diketone, one azo compound, one peroxide, one benzildimethylketal, one benzoin ether, one dialkoxyacetophenone and/or one trimethylbenzoyl phosphine oxide.

9. Dental material according to any one of the preceding claims, which contains as accelerator an amine, a barbituric acid derivative or a sulfinic acid derivative.

10. Dental material according to any one of the preceding claims, which additionally contains at least one UV absorber which has at least one radically polymerisable group.

11. Dental material according to any one of the preceding claims, which additionally contains an antioxidant which has at least one radically polymerisable group.

12. Dental material according to any one of the preceding claims, which additionally contains UV stabiliser and an inhibitor, which both have a radically polymerisable group.

13. Dental material according to any one of the preceding claims, which does not contain any non-radically polymerisable monomeric or polymeric constituents.

14. Dental material according to any one of the preceding claims, which does not contain any solvent.

15. Dental material according to any one of the preceding claims, which additionally contains filler.

16. Dental material according to claim 15, wherein the filler is surface-modified with an adhesion promoter.

17. Dental material according to claim 16, wherein the adhesion promoter has at least one radically polymerisable group.

18. Dental material according to any one of the preceding claims, which additionally contains one or more pigments.

19. Dental material according to any one of the preceding claims, which apart from pigments and filler, contains exclusively components which have radically polymerisable groups.

20. Dental material according to any one of the preceding claims, which contains:
(a) 1 to 50 wt.%, in particular 5 to 40 wt.% or binder,
(b) 0.1 to 5.0 wt.%, in particular 0.2 to 2.0 wt.% of initiator,
(c) 0.1 to 5.0 wt.%, in particular 0.2 to 2.0 wt.% of accelerator,
(d) 0.01 to 3.0 wt.%, in particular 0.05 to 2.0 wt.% of inhibitor,
in each case relative to the total mass of the material.

21. Dental material according to claim 20, which additionally contains (e) 0 to 90, in particular 3 to 80 wt.% of filler, relative to the total mass of the material.

22. Use of a composition, which contains a radically polymerisable organic binder, an initiator for the radical polymerisation, an accelerator for the radical polymerisation and an inhibitor for the radical polymerisation, wherein the initiator, the accelerator and the inhibitor each have at least one radically polymerisable group, as dental material or for production of a dental material.

## Revendications

1. Matériau dentaire contenant un liant organique polymérisable par voie radicalaire, au moins un initiateur pour la polymérisation radicalaire, au moins un activateur pour la polymérisation radicalaire et au moins un inhibiteur pour la polymérisation radicalaire, **caractérisé en ce que** l'initiateur, l'activateur et l'inhibiteur présentent respectivement au moins un groupement polymérisable par voie radicalaire.

2. Matériau dentaire selon la revendication 1, dans lequel le liant contient des monomères et/ou des oligomères ayant deux groupements polymérisables par voie radicalaire ou plus.

3. Matériau dentaire selon la revendication 2, dans lequel le liant contient au moins un polysiloxane ayant des groupements polymérisables par voie radicalaire.

4. Matériau dentaire selon la revendication 2 ou 3, dans lequel le liant contient au moins un dendrimère qui présente des groupements polymérisables par voie radicalaire.

5. Matériau dentaire selon l'une quelconque des revendications 2 à 4, dans lequel le liant contient au moins un monomère de réticulation polymérisable par voie radicalaire.

6. Matériau dentaire selon l'une quelconque des revendications 2 à 5, dans lequel le liant contient exclusivement des monomères polymérisables par voie radicalaire et/ou des oligomères ayant deux groupements polymérisables par voie radicalaire ou plus.

7. Matériau dentaire selon l'une quelconque des revendications précédentes, qui contient un photoinitiateur.

8. Matériau dentaire selon l'une quelconque des revendications précédentes, qui contient comme initiateur au moins une alpha-dicétone, un composé azoïque, un peroxyde, un benzyldiméthylcétal, un éther de benzoïne, une dialcoxyacétophénone et/ou un oxyde de triméthylbenzoylphosphine.

9. Matériau dentaire selon l'une quelconque des revendications précédentes, qui contient comme activateur une amine, un dérivé d'acide barbiturique ou un dérivé d'acide sulfinique.

10. Matériau dentaire selon l'une quelconque des revendications précédentes, qui contient en plus au moins un absorbeur d'UV qui présente au moins un groupement polymérisable par voie radicalaire.

11. Matériau dentaire selon l'une quelconque des revendications précédentes, qui contient en plus un agent antioxydant qui présente au moins un groupement polymérisable par voie radicalaire.

12. Matériau dentaire selon l'une quelconque des revendications précédentes contenant un stabilisateur UV et un inhibiteur qui présentent tous deux un groupement polymérisable par voie radicalaire.

13. Matériau dentaire selon l'une quelconque des revendications précédentes, qui ne contient pas de composants monomères ou polymères non polymérisables par voie radicalaire.

14. Matériau dentaire selon l'une quelconque des revendications précédentes, qui ne contient pas de solvant.

15. Matériau dentaire selon l'une quelconque des revendications précédentes, qui contient en plus une charge.

16. Matériau dentaire selon la revendication 15, dans lequel la charge est modifiée en surface par un agent adhésif.

17. Matériau dentaire selon la revendication 16, dans lequel l'agent adhésif présente au moins un groupement polymérisable par voie radicalaire.

18. Matériau dentaire selon l'une quelconque des revendications précédentes, qui contient en plus un ou plusieurs pigments.

19. Matériau dentaire selon l'une quelconque des revendications précédentes qui contient, en dehors des pigments et de la charge, exclusivement des composants qui présentent des groupements polymérisables par voie radicalaire.

20. Matériau dentaire selon l'une quelconque des revendications précédentes, qui contient :
(a) 1 à 50 % en poids, en particulier 5 à 40 % en poids de liant,
(b) 0,1 à 5,0 % en poids, en particulier 0,2 à 2,0 % en poids d'initiateur,
(c) 0,1 à 5,0 % en poids, en particulier 0,2 à 2,0 % en poids d'activateur,
(d) 0,01 à 3,0 % en poids, en particulier 0,05 à 2,0 % en poids d'inhibiteur,
respectivement, par rapport à la masse totale du matériau.

21. Matériau dentaire selon la revendication 20, qui contient en plus (e) 0 à 90, en particulier 3 à 80 % en poids de charge, par rapport à la masse totale du matériau.

22. Utilisation d'une composition, qui contient un liant organique polymérisable par voie radicalaire, un initiateur pour la polymérisation radicalaire, un activateur pour la polymérisation radicalaire et un inhibiteur pour la polymérisation radicalaire, dans laquelle l'initiateur, l'activateur et l'inhibiteur présentent respectivement au moins un groupement polymérisable par voie radicalaire, comme matériau dentaire ou pour fabriquer un matériau dentaire.
